Europäisches Patentamt
European Patent Office
Office européen des brevets

(11) Veröffentlichungsnummer: **0 276 406**
**A1**

(12) # EUROPÄISCHE PATENTANMELDUNG

(21) Anmeldenummer: **87117670.7**

(22) Anmeldetag: **30.11.87**

(51) Int. Cl.4: **C07D 239/42**, A01N 43/54, C07D 239/30, //C07D239/36, C07D239/56,C07C69/716

(30) Priorität: **12.12.86 DE 3642452**

(43) Veröffentlichungstag der Anmeldung: **03.08.88 Patentblatt 88/31**

(84) Benannte Vertragsstaaten: **AT BE CH DE ES FR GB IT LI NL**

(71) Anmelder: **BAYER AG**

**D-5090 Leverkusen(DE)**

(72) Erfinder: **Jelich, Klaus, Dr.**
**Pahlkestrasse 5**
**D-5600 Wuppertal 1(DE)**
Erfinder: **Dutzmann, Stefan, Dr.**
**Leinenweberweg 33**
**D-4000 Düsseldorf 13(DE)**
Erfinder: **Reinecke, Paul, Dr.**
**Steinstrasse 8**
**D-5090 Leverkusen 3(DE)**
Erfinder: **Brandes, Wilhelm, Dr.**
**Eichendorffstrasse 3**
**D-5653 Leichlingen(DE)**
Erfinder: **Stendel, Wilhelm, Dr.**
**In den Birken 55**
**D-5600 Wuppertal 1(DE)**

(54) **Substituierte 4-Amino-pyrimidine.**

(57) Substituierte 4-Amino-pyrimidine der allgemeinen Formel (I),

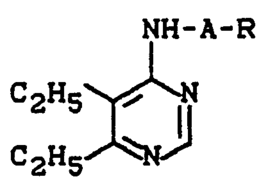

(I)

in welcher
R für jeweils gegebenenfalls substituiertes Phenyl, Phenoxy oder Benzyloxy steht und
A für eine Alkylenkette steht, sowie ihre Verwendung zur Bekämpfung von Schädlingen und das neue 4-Chlor-5,6-diethyl-pyrimidin.

Die neuen substituierten 4-Amino-pyrimidine der allgemeinen Formel (I) können z.B. hergestellt werden, indem man 4-Chlor-5,6-diethyl-pyrimidin mit geeigneten Aminen umsetzt.

Das neue 4-Chlor-5,6-diethyl-pyrimidin kann z.B. hergestellt werden, indem man 2-Mercapto-4-hydroxy-5,6-diethyl-pyrimidin zunächst in einer 1. Stufe mit Wasser stoffperoxid und anschließend mit Kaliumcarbonat in Gegenwart von Wasser als Verdünnungsmittel umsetzt und das so erhältliche 4-Hydroxy-5,6-diethyl-pyrimidin in

EP 0 276 406 A1

einer 2. Stufe mit Phosphoroxychlorid in Gegenwart eines Verdünnungsmittels und gegebenenfalls in Gegenwart eines Säurebindemittels umsetzt.

## Substituierte 4-Amino-pyrimidine

Die Erfindung betrifft neue substituierte 4-Amino-pyrimidine, ein Verfahren zu ihrer Herstellung sowie ihre Verwendung als Schädlingsbekämpfungsmittel.

Es ist bereits bekannt, daß bestimmte substituierte 4-Amino-pyrimidine, wie beispielsweise das 5,6-Dimethyl-4-[2-(4-methylphenoxy)-ethyl]-amino-pyrimidin, gute fungizide, insektizide und akarizide Eigenschaften besitzen (vgl. EP 57 440). Weiterhin ist bekannt, daß Zink-organische Verbindungen, wie beispielsweise das Zink-ethylen-1,2-bis-(dithiocarbamat), insbesondere bei bestimmten pilzlichen Schaderregern gute Wirkung besitzen (vgl. z. B. K.-H. Büchel "Pflanzenschutz und Schädlingsbekämpfung" S. 137 f, Thieme Verlag, Stuttgart 1977).

Die fungizide, insektizide und akarizide Wirksamkeit dieser vorbekannten Verbindungen ist jedoch insbesondere bei niedrigen Aufwandmengen und Konzentrationen nicht in allen Anwendungsbereichen völlig zufriedenstellend.

Es wurden neue substituierte 4-Amino-pyrimidine der allgemeinen Formel (I),

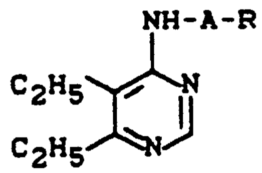

(I)

in welcher
R für jeweils gegebenenfalls substituiertes Phenyl, Phenoxy oder Benzyloxy steht und
A für eine Alkylenkette steht,
gefunden.

Weiterhin wurde gefunden, daß man die neuen substituierten 4-Amino-pyrimidine der allgemeinen Formel (I),

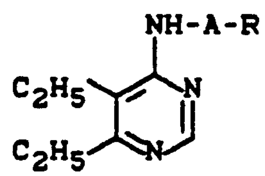

(I)

in welcher
R für jeweils gegebenenfalls substituiertes Phenyl, Phenoxy oder Benzyloxy steht und
A für eine Alkylenkette steht,
erhält, wenn man 4-Chlor-5,6-diethyl-pyrimidin der Formel (II),

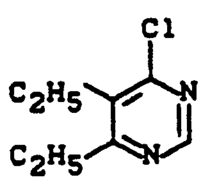

(II)

mit Aminen der Formel (III),
$H_2N$-A-R (III)
in welcher
R und A die oben angegebene Bedeutung haben,
gegebenenfalls in Gegenwart eines Verdünnungsmittels und gegebenenfalls in Gegenwart eines Säurebindemittels umsetzt.

Schließlich wurde gefunden, daß die neuen substituierten 4-Amino-pyrimidine der allgemeinen Formel (I) eine gute Wirkung gegen Schädlinge insbesondere gegen pilzliche Schädlinge und gegen Insekten und Milben besitzen.

Überraschenderweise zeigen die erfindungsgemäßen substituierten 4-Amino-pyrimidine eine erheblich bessere fungizide, insektizide und akarizide Wirksamkeit als die aus dem Stand der Technik bekannten 4-Amino-pyrimidine, wie beispielsweise das 5,6-Dimethyl-4-[2-(4-methylphenoxy)-ethyl]-amino-pyrimidin oder die aus dem Stand der Technik bekannten Zink-organischen Verbindungen, wie beispielsweise das Zink-ethylen-1,2-bis-(dithiocarbamat), welches chemisch und/oder wirkungsmäßig naheliegende Verbindungen sind.

Die erfindungsgemäßen substituierten 4-Amino-pyrimidine sind durch die Formel (I) allgemein definiert. Bevorzugt sind Verbindungen der Formel (I), bei welchen

R für jeweils gegebenenfalls einfach bis mehrfach, gleich oder verschieden im Phenylteil substituiertes Phenyl, Phenoxy oder Benzyloxy steht, wobei als Substituenten jeweils in Frage kommen: Halogen, sowie jeweils geradkettiges oder verzweigtes Alkyl, Alkoxy oder Alkylthio mit jeweils 1 bis 8 Kohlenstoffatomen und

A für eine geradkettige oder verzweigte Alkylenkette mit 1 bis 8 Kohlenstoffatomen steht.

Halogen steht hierbei im allgemeinen für Fluor, Chlor, Brom oder Jod.

Alkyl steht hierbei im allgemeinen für einen geradkettigen oder verzweigten Kohlenwasserstoffrest mit 1 bis 8 Kohlenstoffatomen. Besonders bevorzugt wird Niedrigalkyl mit 1 bis etwa 4 Kohlenstoffatomen. Genannt seien beispielsweise Methyl, Ethyl, Propyl, Isopropyl, Butyl, Isobutyl, Pentyl, Isopentyl, Hexyl, Isohexyl, Heptyl, Isoheptyl, Octyl und Isooctyl.

Alkoxy steht im allgemeinen für einen über ein Sauerstoffatom gebundenen geradkettigen oder verzweigten Kohlenwasserstoffrest mit 1 bis 8 Kohlenstoffatomen. Besonders bevorzugt ist ein Alkoxyrest mit 1 bis 3 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

Alkylthio steht im allgemeinen für einen über ein Schwefelatom gebundenen, geradkettigen oder verzweigten Kohlenwasserstoffrest mit 1 bis 8 Kohlenstoffatomen.

Besonders bevorzugt ist ein Alkylthiorest mit 1 oder 2 Kohlenstoffatomen. Beispielsweise seien Methylthio, Ethylthio, Propylthio, Isopropylthio, Butylthio, Isobutylthio, Pentylthio, Isopentylthio, Hexylthio, Isohexylthio, Heptylthio, Isoheptylthio, Octylthio und Isooctylthio genannt.

Alkylen steht im allgemeinen für einen geradkettigen oder verzweigten Kohlenwasserstoffrest mit 1 bis 8 Kohlenstoffatomen, besonders bevorzugt wird Alkylen mit 1 bis 4 Kohlenstoffatomen. Genannt seien beispielsweise Methylen, Ethylen, Propylen, iso-Propylen, n-Butylen, iso-Butylen, sec.-Butylen, Pentylen, iso-Pentylen, Hexylen, iso-Hexylen, Heptylen, iso-Heptylen, Octylen und iso-Octylen.

Besonders bevorzugt sind Verbindungen der Formel (I) bei welchen

R für jeweils gegebenenfalls ein-bis dreifach, gleich oder verschieden im Phenylteil substituiertes Phenyl, Phenoxy oder Benzyloxy steht, wobei als Substituenten jeweils in Frage kommen: Fluor, Chlor, Brom, Iod, Methyl, Ethyl, n-oder i-Propyl, n-, i-, s-oder t-Butyl, Methoxy, Ethoxy, n-oder i-Propoxy, Methylthio oder Ethylthio und

A für eine geradkettige oder verzweigte Alkylenkette mit 1 bis 4 Kohlenstoffatomen steht.

Im einzelnen seien die bei den Herstellungsbeispielen genannten Verbindungen der allgemeinen Formel (I) genannt.

Verwendet man beispielsweise 4-Chlor-5,6-diethyl-pyrimidin und 2-(2,4-Dimethylphenoxy)-ethylamin als Ausgangsstoffe, so läßt sich der Reaktionsablauf des erfindungsgemäßen Verfahrens durch das folgende Formelschema darstellen:

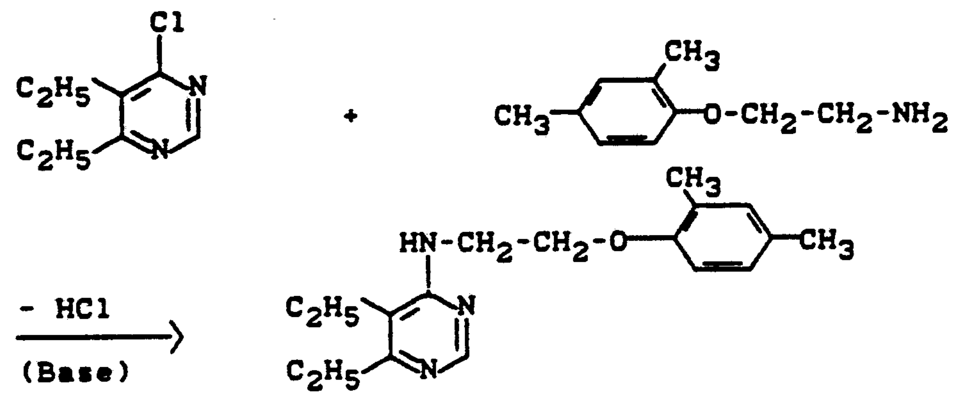


Das zur Durchführung des erfindungsgemäßen Verfahrens als Ausgangsverbindung benötigte 4-Chlor-5,6-diethyl-pyrimidin der Formel (II) ist noch nicht bekannt.

Man erhält es, wenn man 2-Mercapto-4-hydroxy-5,6-diethyl-pyrimidin (vgl. Rocz. Chem. 48, 971 - 980 [1974] bzw. CA 81: 169505b) der Formel (IV)

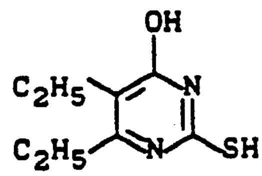

(IV)

zunächst in einer 1. Stufe mit Wasserstoffperoxid und anschließend mit Kaliumcarbonat in Gegenwart von Wasser als Verdünnungsmittel bei Temperaturen zwischen 50 ° C und 120 ° C umsetzt und das so erhältliche 4-Hydroxy-5,6-diethyl-pyrimidin der Formel (V)

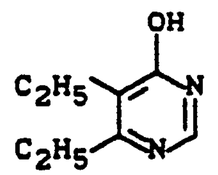

(V)

in einer 2. Stufe mit Phosphoroxychlorid in Gegenwart eines Verdünnungsmittels wie beispielsweise Chloroform und gegebenenfalls in Gegenwart eines Säurebindemittels wie beispielsweise Triethylamin bei Temperaturen zwischen 20 ° C und 80 ° C umsetzt.

Die zur Durchführung des erfindungsgemäßen Verfahrens weiterhin als Ausgangsstoffe benötigten Amine sind durch die Formel (III) allgemein definiert. In dieser Formel (III) stehen R und A für diejenigen Reste, die bereits im Zusammenhang mit der Beschreibung der erfindungsgemäßen Stoffe der Formel (I) für diese Substituenten genannt wurden.

Die Amine der Formel (III) sind allgemein bekannte Verbindungen der organischen Chemie oder erhältlich in Analogie zu allgemein bekannten Verfahren.

Als Verdünnungsmittel zur Durchführung des erfindungsgemäßen Verfahrens kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl-oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester oder Sulfoxide, wie Dimethylsulfoxid. Es ist auch möglich, das erfindungsgemäße Verfahren ohne zusätzliche Verdünnungsmittel "in Substanz" durchzuführen.

Das erfindungsgemäße Verfahren wird gegebenenfalls in Gegenwart eines geeigneten Säurebindemittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid; Alkalimetallcarbonate, wie Natriumcarbonat, Kaliumcarbonat oder Natriumhydrogencarbonat; sowie tertiäre Amine, wie Triethylamin, N,N-Dimethylanilin, Pyridin, N,N-Dimethylaminopyridin, Diazabicyclooctan (DABCO), Diazabicyclononen(DBN) oder Diazabicycloundecen(DBU).

Es ist auch möglich, das als Reaktionspartner in Frage kommende Amin der Formel (III) in entsprechendem Über schuß gleichzeitig als Säurebindemittel einzusetzen.

Die Reaktionstemperaturen können bei der Durchführung des erfindungsgemäßen Verfahrens in einem größeren Bereich variiert werden. Im allgemeinen arbeitet man bei Temperaturen zwischen 20 ° C und 250 ° C, vorzugsweise bei Temperaturen zwischen 50 ° C und 200 ° C.

Zur Durchführung des erfindungsgemäßen Verfahrens setzt man pro Mol an 4-Chlor-5,6-diethylpyrimidin der Formel (II) im allgemeinen 1,0 bis 5,0 Mol, vorzugsweise 1,0 bis 2,0 Mol an Amin der Formel (III) und gegebenenfalls 1,0 bis 2,0 Mol an Säurebindemittel ein.

Die Reaktionsdurchführung, Aufarbeitung und Isolierung der Reaktionsprodukte der Formel (I) erfolgt nach üblichen Methoden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind z.B. für

den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanzen-und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des Braunrostes an Getreide (Puccinia recondita), gegen den Erreger des echten Getreidemehltaus (Erysiphe graminis), gegen den Erreger der Reisfleckenkrankheit (Pyricularia oryzae) sowie zur Bekämpfung von Krankheiten im Gemüsebau, wie beispielsweise gegen den Erreger der Tomatenbraunfäule (Phytophthora infestans) einsetzen.

Darüber hinaus eignen sich die Wirkstoffe zur Bekämpfung von tierischen Schädlingen, vorzugsweise Arthropoden, insbesondere Insekten und Spinnentieren, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Ohniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpha spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrospiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monolmorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Zenopsylla cheopis, Ceratophyllus spp.,

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe wirken nicht nur gegen Pflanzen-, Hygiene-und Vorratsschädlinge, sondern auch auf dem veterinär-medizinischen Sektor bei Nutztieren, wie z.B. Rindern, Schafen, Schweinen, Pferden, Ziegen, Büffeln, Kamelen; bei Hobbytieren, wie z.B. Hunden, Katzen; bei zog. Labortieren, wie Ratten, Mäusen, Hamstern, Kaninchen sowie Bienen gegen tierische Parasiten (Ektoparasiten), wie Schildzecken, Lederzekken, Räudemilben, Laufmilben, Fliegen (stechend und lekkend), parasitierende Fliegenlarven, Läuse, Haarlinge, Federlinge, Flöhe. Sie sind gegen normalsensible und resistente Arten und Stämme, sowie auf alle parasitierende und nichtparasitierende Entwicklungsstadien der Ektoparasiten wirksam. Insbesondere eignen sich die erfindungsgemäßen Wirkstoffe auf diesem Gebiet zur Bekämpfung von Räudemilben (Psoroptes ovis).

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Armotaten oder chlorierte aliphatische Kohlenwasserstoffe, wie Chlorbenzole, Chlorethylene

oder Methylenchlorid, aliphatische Kohlenwasserstoffe, wie Cyclohexan oder Paraffine, z.B. Erdölfraktionen. Alkohole, wie Butanol oder Glycol sowie deren Ether und Ester, Ketone, wie Aceton. Methylethylketon. Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Ge steine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder -schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Verwendung als Fungizide in den Formulierungen in Mischung mit anderen bekannten Wirkstoffen vorliegen wie Fungiziden, Bakteriziden, Insektiziden, Akariziden, Herbiziden, Schutzstoffen gegne Vogelfraß, Wuchsstoffen, Pflanzennährstoffen und Bodenstrukturverbesserungesmitteln.

Die Wirkstoffe können als solche, in Form ihrer Formulierungen oder den daraus bereiteten Anwendungsformen wie gebrauchsfertige Lösungen, Suspensionen, SPritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Bei der Verwendung als Insektizide und Akarizide können die erfindungsgemäßen Wirkstoffe ebenfalls in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßem Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene-und Vorratsschädlinge zeichnen sich die Wirkstoffe durch eine

hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z. B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Herstellungsbeispiele

Beispiel 1

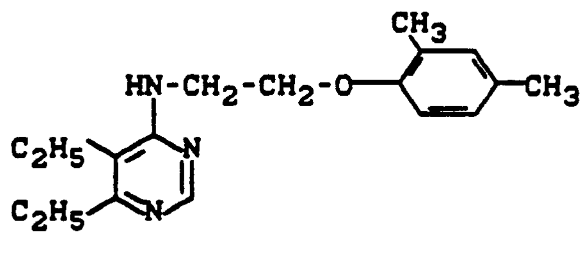

Eine Mischung aus 8,0 g (0,047 Mol) 4-Chlor-5,6-diethyl-pyrimidin und 15,6 g (0,094 Mol) 2-(2,4-Dimethyl-phenoxy)-ethylamin (vgl. z. B. EP 103 114) wird 2 Stunden lang auf 150 ° C erhitzt. Zur Aufarbeitung nimmt man die erkaltete Reaktionsmischung in Wasser auf, extrahiert dreimal mit Dichlormethan, wäscht die vereinigten organischen Phasen mehrmals mit Wasser, trocknet über Natriumsulfat, engt im Vakuum ein und reinigt den Rückstand chromatographisch (Kieselgel/Ether).

Man erhält 7,5 g (53,4 % der Theorie) an 5,6-Diethyl-4-[2-(2,4-dimethylphenoxy)-ethylamino]-pyrimidin vom Schmelzpunkt 79 ° C.

Herstellung der Ausgangsverbindung

1a

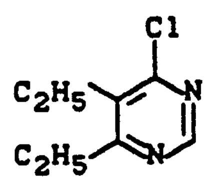

Zu 17 g (0,112 Mol) 4-Hydroxy-5,6-diethylpyrimidin in 100 ml Chloroform gibt man zuerst 11,3 g (0,112 Mol) Triethylamin und tropft anschließend 54,9 g (0,358 Mol) Phosphoroxychlorid zu, wobei sich das Gemisch auf Rückflußtemperatur erhitzt. Nach beendeter Zugabe rührt man weitere 60 Minuten bei Rückflußtemperatur, engt dann im Vakuum ein, gießt den Rückstand in Eiswasser, gibt portionsweise Kaliumcarbonat zu bis zur alkalischen Reaktion, extrahiert mehrfach mit Chloroform, trocknet über Natriumsulfat, engt im Vakuum ein und destilliert den Rückstand im Wasserstrahlvakuum.

Man erhält 15,0 g (79 % der Theorie) an 4-Chlor-5,6-diethyl-pyrimidin vom Siedepunkt 100 ° C bei 18 mbar.

**1a1**

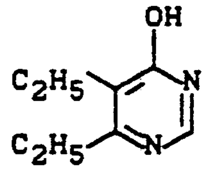

Zu 300 ml 12-prozentigem wässrigem Wasserstoffperoxid gibt man bei 80 ° C portionsweise 28 g (0,152 Mol) 2-Mercapto-4-hydroxy-5,6-diethylpyrimidin, so daß die Temperatur ohne zusätzliche Heizung bei 80 ° C bleibt. Nach beendeter Zugabe engt man die erkaltete Reaktionsmischung auf etwa ein Drittel des Volumens ein, gibt portionsweise Kaliumcarbonat zu bis zur alkalischen Reaktion, extrahiert mehrmals mit Chloroform, trocknet über Natriumsulfat und entfernt das Lösungsmittel im Vakuum.
Man erhält 20,1 g (87 % der Theorie) an 4-Hydroxy-5,6-diethyl-pyrimidin vom Schmelzpunkt 130 ° C.

**1a2**

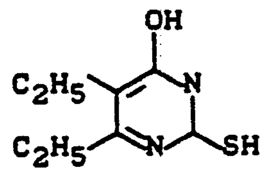

Zu einer frisch hergestellten, noch heißen Lösung von 6,2 g (0,269 Mol) Natrium in 250 ml absolutem Ethanol läßt man 46,0 g (0,267 Mol) α-Propionyl-buttersäureethylester fließen, gibt anschließend 20,35 g (0,267 Mol) Thioharnstoff zu und erhitzt für weitere 2-Stunden auf Rückflußtemperatur. Zur Aufarbeitung engt man im Vakuum ein, nimmt den Rückstand in Wasser auf und fällt das Produkt durch Zugabe von 28 ml konzentrierter Salzsäure aus.
Nach Absaugen und Trocknen erhält man 28,7 g (58 % der Theorie) an 2-Mercapto-4-hydroxy-5,6-diethyl-pyrimidin vom Schmelzpunkt 192 ° C.

**1a3**

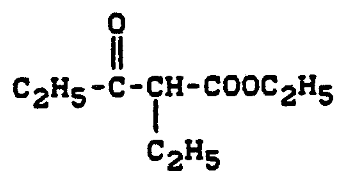

Zu einer frisch hergestellten noch heißen Lösung aus 13 g (0,565 Mol) Natrium in 250 ml absolutem Ethanol läßt man 81,5 g (0,565 Mol) Propionylessigsäureethylester fließen, gibt anschließend 88,1 g (0,565 Mol) Ethyliodid zu und erhitzt 3 Stunden auf Rückflußtempera tur. Zur Zufarbeitung engt man im Vakuum ein, nimmt den Rückstand in Wasser auf, extrahiert mehrfach mit Ether, trocknet die vereinigten organischen Phasen über Natriumsulfat, engt ein und destilliert den Rückstand im Wasserstrahlvakuum.
Man erhält 84,1 g (87 % der Theorie) an α-Propionylbuttersäureethylester vom Siedepunkt 88 ° C bei 18 mbar.

In entsprechender Weise und gemäß den allgemeinen Angaben zur Herstellung erhält man die folgenden substituierten 4-Amino-pyrimidine der allgemeinen Formel (I):

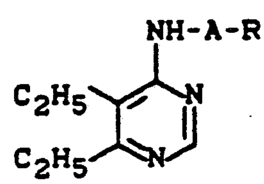

(I)

| Bsp.-Nr. | A | R | Schmelzpunkt [°C] |
|---|---|---|---|
| 2 | $-CH_2-CH_2-$ | 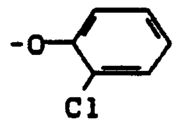 | 112 - 115 |
| 3 | $-CH_2-CH_2-$ | 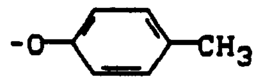 | 89 - 90 |
| 4 | $-CH_2-CH_2-$ | 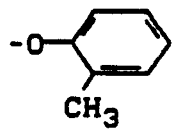 | 98 - 100 |
| 5 | $-CH_2-CH_2-$ | 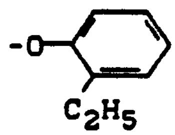 | Öl<br>$^1$H-NMR*): 3,95 |
| 6 | $-CH_2-CH_2-$ | 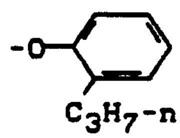 | Öl<br>$^1$H-NMR*): 3,95 |
| 7 | 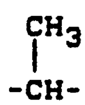 | 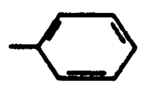 | 73 - 76 |

*) Die $^1$H-NMR-Spektren wurden in $CDCl_3$ mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurden die nachstehend aufgeführten Verbindungen als Vergleichssubstanzen eingesetzt:

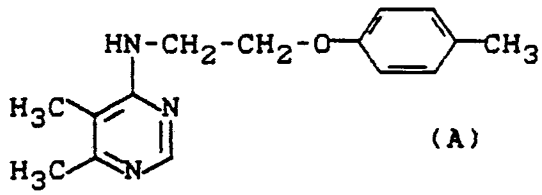

(A)

5,6-Dimethyl-4-[2-(4-methylphenoxy)-ethyl]-amino-pyrimidin (bekannt aus EP 57 440) und

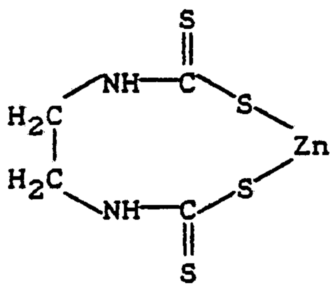

(B)

Zink-ethylen-1,2-bis-(dithiocarbamat) (bekannt aus K.-H. Büchel "Pflanzenschutz und Schädlingsbekämpfung" S. 137 f, Thieme Verlag, Stuttgart, 1977).

Beispiel A

Puccinia-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20 ° C und 100 % rel. Luftfeuchtigkeit in einer Inkubationskabine.

Die Pflanzen werden in einem Gewächshaus bei einer Temperatur von ca. 20 ° C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigt bei diesem Test z. B. die Verbindung gemäß Herstellungsbeispiel: (1)

Beispiel B

Phytophthora-Test (Tomate) kurativ

Lösungsmittel: 4.7 Gewichtsteile Aceton
Emulgator: 0.3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20 ° C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100 % relativer Luftfeuchtigkeit und ca. 20 ° C aufgestellt.

3 Tage nach der Inokulation erfolgt die Auswertung.

Eine deutliche Überlegenheit in der Wirksamkeit gegenüber dem Stand der Technik zeigt in diesem Test z. B. die Verbindung gemäß Herstellungsbeispiel: (1)

Beispiel C

Test mit Psoroptes ovis

Lösungsmittel: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

Etwa 10 - 25 Psoroptes ovis werden in 1 ml der zu testenden Wirkstoffzubereitung gebracht, die in Tablettennester einer Tiefziehverpackung pipettiert wurden. Nach 24 Stunden wird der Abtötungsgrad bestimmt.

Bei diesem Test zeigt z. B. die Verbindung gemäß dem Herstellungsbeispiel 1 überlegene Wirkung gegenüber dem Stand der Technik.

**Ansprüche**

1. Substituierte 4-Amino-pyrimidine der allgemeinen Formel (I),

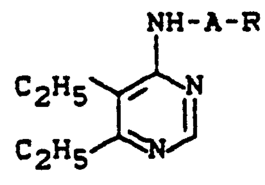

(I)

in welcher
R für jeweils gegebenenfalls substituiertes Phenyl, Phenoxy oder Benzyloxy steht und
A für eine Alkylenkette steht.

2. Substituierte 4-Amino-pyrimidine der Formel (I) gemäß Anspruch 1, bei welchen
R für jeweils gegebenenfalls einfach bis mehrfach, gleich oder verschieden im Phenylteil substituiertes Phenyl, Phenoxy oder Benzyloxy steht, wobei als Substituenten genannt seien: Halogen, sowie jeweils geradkettiges oder verzweigtes Alkyl, Alkoxy oder Alkylthio mit jeweils 1 bis 8 Kohlenstoffatomen und
A für eine geradkettige oder verzweigte Alkylenkette mit 1 bis 8 Kohlenstoffatomen steht.

3. Substituierte 4-Amino-pyrimidine der Formel (I) gemäß Anspruch 1, bei welchen
R für jeweils gegebenenfalls ein-bis dreifach, gleich oder verschieden im Phenylteil substituiertes Phenyl, Phenoxy oder Benzyloxy steht, wobei als Substituenten jeweils in Frage kommen: Fluor, Chlor, Brom, Iod,

Methyl, Ethyl, n-oder i-Propyl, n-, i-, s-oder t-Butyl, Methoxy, Ethoxy, n-oder i-Propoxy, Methylthio oder Ethylthio oder
A für eine geradkettige oder verzweigte Alkylenkette mit 1 bis 4 Kohlenstoffatomen steht.

4. Verfahren zur Herstellung von substituierten 4-Amino-pyrimidinen der allgemeinen Formel (I),

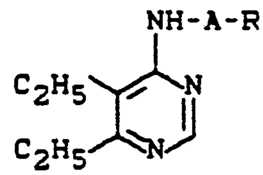

(I)

in welcher
R für jeweils gegebenenfalls substituiertes Phenyl, Phenoxy oder Benzyloxy steht und
A für eine Alkylenkette steht,
dadurch gekennzeichnet, daß man 4-Chlor-5,6-diethyl-pyrimidin der Formel (II),

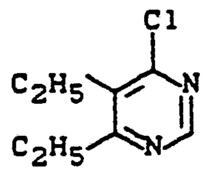

(II)

mit Aminen der Formel (III),
$H_2N$-A-R (III)
in welcher
R und A die oben angegebene Bedeutung haben,
gegebenenfalls in Gegenwart eines Verdünnungsmittels und gegebenenfalls in Gegenwart eines Säurebindemittels umsetzt.

5. Schädlingsbekämpfungsmittel, gekennzeichnet durch einen Gehalt an mindestens einem substituierten 4-Amino-pyrimidin der Formel (II) gemäß den Ansprüchen 1 und 4.

6. Verwendung von substituierten 4-Amino-pyrimidinen der Formel (I) gemäß den Ansprüchen 1 und 4 zur Bekämpfung von Schädlingen.

7. Verfahren zur Bekämpfung von Schädlingen, dadurch gekennzeichnet, daß man substituierte 4-Amino-pyrimidine der Formel (I) gemäß den Ansprüchen 1 und 4 auf Schädlinge oder ihren Lebensraum einwirken läßt.

8. Verfahren zur Herstellung von Schädlingsbekämpfungsmitteln, dadurch gekennzeichnet, daß man substituierte 4-Amino-pyrimidine der Formel (I) gemäß den Ansprüchen 1 und 4 mit Streckmitteln und/oder oberflächenaktiven Mitteln vermischt.

9. 4-Chlor-5,6-diethyl-pyrimidin der Formel (II)

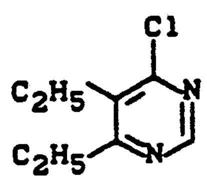

(II).

10. Verfahren zur Herstellung von 4-Chlor-5,6-diethyl-pyrimidin der Formel (II)

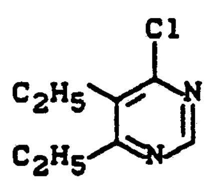

(II)

dadurch gekennzeichnet, daß man 2-Mercapto-4-hydroxy-5,6-diethyl-pyrimidin der Formel (IV)

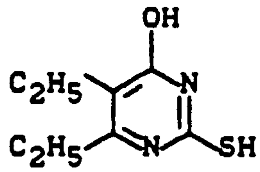

(IV)

zunächst in einer 1. Stufe mit Wasserstoffperoxid und anschließend mit Kaliumcarbonat in Gegenwart eines Verdünnungsmittels bei Temperaturen zwischen 50 ° C und 120 ° C umsetzt und das so erhältliche 4-Hydroxy-5,6-diethyl-pyrimidin der Formel (V)

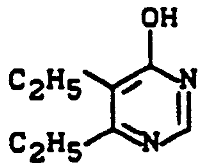

(V)

in einer 2. Stufe mit Phosphoroxychlorid in Gegenwart eines Verdünnungsmittels und gegebenenfalls in Gegenwart eines Säurebindemittels bei Temperaturen zwischen 20 ° C und 80 ° C umsetzt.

Europäisches Patentamt

# EUROPÄISCHER RECHERCHENBERICHT

Nummer der Anmeldung

EP 87 11 7670

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 196 524 (SANKYO COMPANY LTD.) * Verbindungen Nrn. 94,95,96; Tabelle 1, Seite 31; Seite 116; Tabelle 12, Seite 131; Tabelle 13, Seite 144; Tabelle 15, Seite 162; Tabelle 16, Seite 175; Ansprüche 1-3, 5-13, 19-20 * --- | 1-9 | C 07 D 239/42<br>A 01 N 43/54<br>C 07 D 239/30 //<br>C 07 D 239/36<br>C 07 D 239/56<br>C 07 C 69/716 |
| A,D | EP-A-0 057 440 (SANKYO COMPANY LTD.) * Seite 12, Verbindungen 26-29; Ansprüche 1, 7-11, 13-23 * --- | 1-9 | |
| A | PATENT ABSTRACTS OF JAPAN, Band 8, Nr. 126 (C-228)[1563], 13. Juni 1984; & JP - A - 59 36666 (SANKYO K.K.) 28-02-1984 --- | 1,4-9 | |
| A | PATENT ABSTRACTS OF JAPAN, Band 8, Nr. 126 (C-228)[1563], 13. Juni 1984; & JP - A - 59 36667 (SANKYO K.K) 28-02-1984 --- | 1,4-9 | |
| A,D | EP-A-0 103 114 (SANKYO COMPANY LTD) --- | | |
| A | FR-A-2 393 531 (SANKYO COMPANY LTD) ----- | | RECHERCHIERTE SACHGEBIETE (Int. Cl.4)<br>C 07 D 239/00<br>A 01 N 43/00 |

Der vorliegende Recherchenbericht wurde für alle Patentansprüche erstellt

| Recherchenort | Abschlußdatum der Recherche | Prüfer |
|---|---|---|
| BERLIN | 23-03-1988 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)